(19) Europäisches Patentamt
European Patent Office
Office européen des brevets

(11) Publication number: 0 118 838 A2

# (12) EUROPEAN PATENT APPLICATION

(21) Application number: 84102151.2

(22) Date of filing: 01.03.84

(51) Int. Cl.³: C 07 D 501/36
A 61 K 31/545

(30) Priority: 04.03.83 GB 8306061
25.04.83 GB 8311214

(43) Date of publication of application: 19.09.84 Bulletin 84/38

(84) Designated Contracting States: AT BE CH DE FR GB IT LI LU NL SE

(71) Applicant: Fujisawa Pharmaceutical Co., Ltd.
3, Doshomachi 4-chome Higashi-ku
Osaka-shi, Osaka 541(JP)

(72) Inventor: Teraji, Tsutomu
No. 6-20-6 Kofudai Toyono-cho
Toyono-gun Osaka-fu(JP)

(72) Inventor: Sakane, Kazuo
No. 15, Azayamagata
Mino Kawanishi(JP)

(72) Inventor: Goto, Jiro
A-401 Senridai-sukai-taun No. 21
Kashikiriyama Suita(JP)

(74) Representative: Türk, Dietmar, Dr. rer. nat. et al,
Redies, Redies, Türk & Gille Patentanwälte
Brucknerstrasse 20
D-4000 Düsseldorf 13(DE)

(54) New cephem compounds for preparation thereof and pharmaceutical compositions containing them.

(57) A compound of the formula :

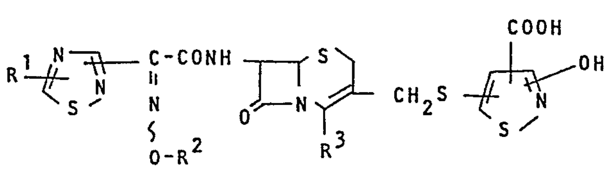

wherein $R^1$ is amino or a protected amino,
$R^2$ is lower alkyl, carboxy (lower) alkyl, protected carboxy (lower) alkyl or cycloalkyl, and
$R^3$ is carboxy or a protected carboxy,
and pharmaceutically acceptable salts thereof, processes for preparation thereof and pharmaceutical compositions containing them in association with a pharmaceutically acceptable, substantially non-toxic carrier or excipient.

EP 0 118 838 A2

0118838

# NEW CEPHEM COMPOUNDS, PROCESSES FOR PREPARATION THEREOF AND PHARMACEUTICAL COMPOSITIONS CONTAINING THEM

The present invention relates to new cephem compounds and pharmaceutically acceptable salts thereof. More particularly, it relates to new cephem compounds and pharmaceutically acceptable salts thereof, which have antimicrobial activities, to processes for preparation thereof, to pharmaceutical composition comprising the same, and to a method of using the same therapeutically in the treatment of infectious diseases in human being and animals.

Accordingly, it is one object of the present invention to provide new cephem compounds and pharmaceutically acceptable salts thereof, which are active against a number of pathogenic microorganisms.

Another object of the present invention is to provide processes for the preparation of new cephem compounds and pharmaceutically acceptable salts thereof.

A further object of the present invention is to provide pharmaceutical composition comprising, as

active ingredients, said new cephem compounds and pharmaceutically acceptable salts thereof.

Still further object of the present invention is to provide a method for the treatment of infectious diseases caused by pathogenic bacteria in human being and animals.

The object new cephem compounds are novel and can be represented by the following general formula :

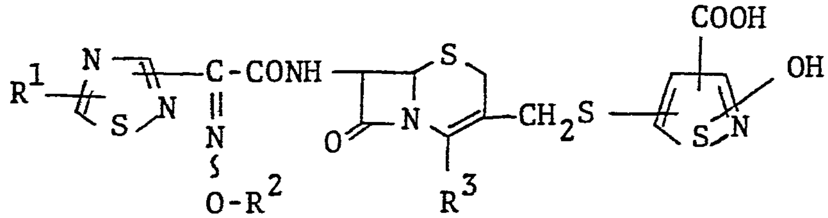

(I)

wherein $R^1$ is amino or a protected amino,

$R^2$ is lower alkyl, carboxy(lower)alkyl, protected carboxy(lower)alkyl or cycloalkyl, and

$R^3$ is carboxy or a protected carboxy.

According to the present invention, the new cephem compound (I) can be prepared by processes which are illustrated in the following schemes.

<u>Process 1</u>

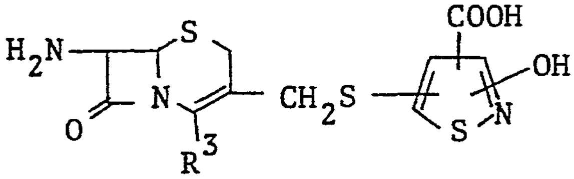

(II)

or its reactive derivative at the amino group or a salt thereof

+

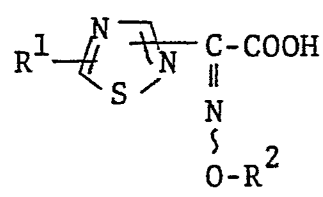

(III)

or its reactive derivative at the carboxy group or a salt thereof

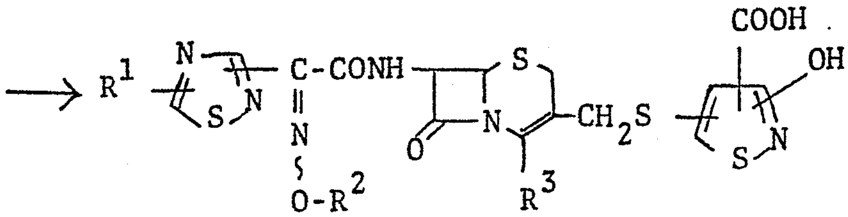

(I)

or a salt thereof

Process 2

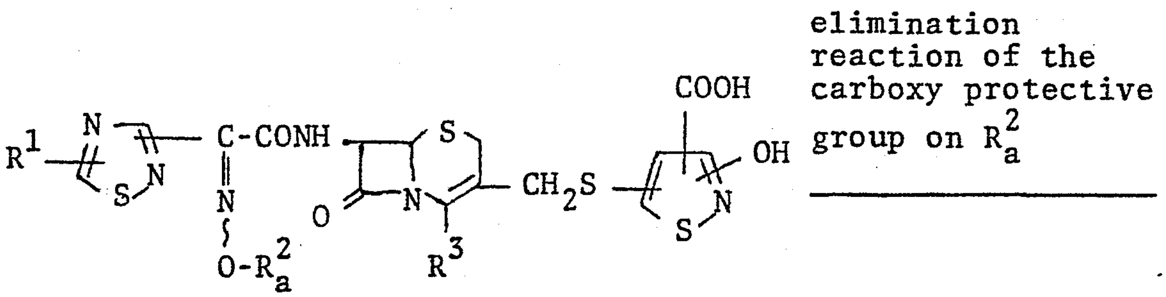


(Ia)

or a salt thereof

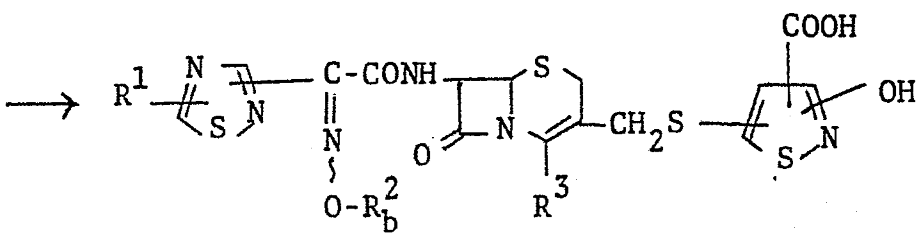

(Ib)

or a salt thereof

wherein $R^1$, $R^2$ and $R^3$ are each as defined above,

$R_a^2$ is protected carboxy(lower)alkyl, and

$R_b^2$ is carboxy(lower)alkyl.

In the present invention, with regard to the object compounds (I) and (Ib) and the starting compounds (III) and (Ia), it is to be understood that all of said

compounds include syn isomer, anti isomer and a mixture thereof. And, as to the object compound (I), the syn isomer thereof means one geometrical isomer having the group represented by the following formula :

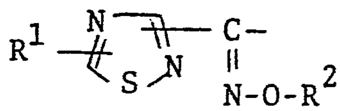

(wherein $R^1$ and $R^2$ are each as defined above) and the anti isomer means the other geometrical isomer having the group of the formula :

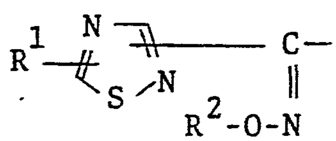

(wherein $R^1$ and $R^2$ are each as defined above).

Further, as to the other compounds, the syn and anti isomers thereof also are represented by the same geometrical configuration as that of the object compound (I), respectively.

Suitable pharmaceutically acceptable salts of the object compound (I) are conventional non-toxic salts and may include an inorganic salt, for example, a metal salt such as an alkali metal salt (e.g., sodium salt, potassium salt, etc.) and an alkaline earth metal salt (e.g., calcium salt, magnesium salt, etc.), ammonium salt etc.; an organic salt, for example, an organic amine salt (e.g., trimethylamine salt, triethylamine salt, pyridine salt, procaine salt, picoline salt, dicyclohexylamine salt, N,N'-dibenzylethylene-diamine salt, N-methylglucamine salt, diethanolamine salt, triethanolamine salt, tris(hydroxymethylamino)methane salt, phenylethylbenzylamine

salt, dibenzylethylenediamine salt, etc.) etc.; an organic carboxylic or sulfonic acid salt (e.g., formate, acetate, maleate, tartrate, methanesulfonate, benzenesulfonate, toluenesulfonate, etc.); an inorganic acid salt (e.g., hydrochloride, hydrobromide, sulfate, phosphate, etc.);
a salt with a basic or acidic amino acid (e.g., arginine, aspartic acid, glutamic acid, lysine, etc.) and the like.

In the above and subsequent descriptions of the present specification, suitable examples and illustration of the various definitions which the present invention intends to include within the scope thereof are explained in detail as follows.

The term "lower" is used to intend a group having 1 to 6 carbon atom(s), unless otherwise provided.

Suitable protected amino may include an acylamino and amino group substituted by a conventional protective group other than the acyl group, such as ar(lower)alkyl(e.g., benzyl, trityl, etc.), ar(lower)-alkylidene(e.g., benzylidene, etc.), lower alkylidene substituted with lower alkoxycarbonyl or di(lower)-alkylamino(e.g., 1-ethoxycarbonyl-2-propylidene, dimethylaminomethylene, etc.) or the like.

Suitable acyl moiety in the term "acylamino" may include carbamoyl, aliphatic acyl group and acyl group containing an aromatic or heterocyclic ring. And, suitable examples of the said acyl may be lower alkanoyl (e.g., formyl, acetyl, propionyl, butyryl, isobutyryl, valeryl, isovaleryl, oxalyl, succinyl, pivaloyl, etc.), preferably one having 1 to 4 carbon atom(s), more preferably one having 1 to 2 carbon atom(s);
lower alkoxycarbonyl having 2 to 7 carbon atoms (e.g., methoxycarbonyl, ethoxycarbonyl, propoxycarbonyl, 1-cyclopropylethoxycarbonyl, isopropoxycarbonyl, butoxycarbonyl, t-butoxycarbonyl, pentyloxycarbonyl,

t-pentyloxycarbonyl, hexyloxycarbonyl, etc.), preferably one having 3 to 6 carbon atoms;
lower alkanesulfonyl (e.g., mesyl, ethanesulfonyl, propanesulfonyl, isopropanesulfonyl, butanesulfonyl, etc.);
arenesulfonyl (e.g., benzenesulfonyl, tosyl, etc.);
aroyl (e.g., benzoyl, toluoyl, naphthoyl, phthaloyl, indancarbonyl, etc.);
ar(lower)alkanoyl (e.g., phenylacetyl, phenylpropionyl, etc.); cyclo(lower)alkyl(lower)alkanoyl(e.g. cyclohexylacetyl, cyclopentylacetyl, etc.);
ar(lower)alkoxycarbonyl (e.g. benzyloxycarbonyl, phenethyloxycarbonyl, etc.); and the like.

Suitable lower alkyl and lower alkyl moiety in the terms "carboxy(lower)alkyl" and "protected carboxy-(lower)alkyl" may include straight or branched one having 1 to 6 carbon atom(s), such as methyl, ethyl, propyl, isopropyl, butyl, isobutyl, sec-butyl, tert-butyl, pentyl, tert-pentyl, hexyl or the like, preferably one having 1 to 4 carbon atom(s).

Suitable cycloalkyl may include one having 3 to 8 carbon atoms, for example, cyclopropyl, cyclobutyl, cyclopentyl, cyclohexyl, cycloheptyl, or the like, preferably one having 4 to 7 carbon atoms.

Suitable protected carboxy and protected carboxy moiety in the term "protected carboxy(lower)alkyl" may include esterified carboxy in which said ester may be the ones such as lower alkyl ester (e.g., methyl ester, ethyl ester, propyl ester, isopropyl ester, butyl ester, isobutyl ester, t-butyl ester, pentyl ester, t-pentyl ester, hexyl ester, 1-cyclopropylethyl ester, etc.), wherein lower alkyl moiety may be preferably one having 1 to 4 carbon atom(s); lower alkenyl ester (e.g., vinyl ester, allyl ester, etc.); lower alkynyl ester (e.g. ethynyl ester, propynyl ester, etc.);

mono(or di or tri)-halo(lower)alkyl ester (e.g., 2-iodoethyl ester, 2,2,2-trichloroethyl ester, etc.); lower alkanoyloxy(lower)alkyl ester (e.g., acetoxymethyl ester, propionyloxymethyl ester, 1-acetoxypropyl ester, valeryloxymethyl ester, pivaloyloxymethyl ester, hexanoyloxymethyl ester, 1-acetoxyethyl ester, 2-propionyloxyethyl ester, 1-isobutyryloxyethyl ester, etc.); lower alkanesulfonyl(lower)alkyl ester (e.g., mesylmethyl ester, 2-mesylethyl ester etc.); ar(lower)alkyl ester, for example, phenyl(lower)alkyl ester which may be substituted with one or more suitable substituent(s) (e.g., benzyl ester, 4-methoxybenzyl ester, 4-nitrobenzyl ester, phenethyl ester, trityl ester, diphenylmethyl ester, bis(methoxyphenyl)methyl ester, 3,4-dimethoxybenzyl ester, 4-hydroxy-3,5-ditertiarybutylbenzyl ester, etc.); lower alkoxycarbonyloxy(lower)alkyl ester (e.g., methoxycarbonyloxymethyl ester, ethoxycarbonyloxymethyl ester, ethoxycarbonyloxyethyl ester, etc.) which may be substituted with azido; a heterocyclic ester, preferably benzotetrahydrofuryl ester which may be substituted with oxo group, more preferably phthalidyl ester; aroyloxy(lower)alkyl ester (e.g., benzoyloxymethyl ester, benzoyloxyethyl ester, toluoyloxyethyl ester, etc.); aryl ester which may have one or more suitable substituent(s) (e.g., phenyl ester, tolyl ester, tertiarybutylphenyl ester, xylyl ester, mesityl ester, cumenyl ester, etc.), and the like.

Preferred embodiments of the object compound (I) are as follows.

Preferred embodiment of $R^1$ is amino;

$R^2$ is lower alkyl, carboxy(lower)alkyl, protected carboxy(lower)alkyl [more preferably esterified carboxy(lower)alkyl, most preferably lower

alkoxycarbonyl(lower)alkyl] or cyclo(lower)-alkyl; and

$R^3$ is carboxy.

The processes for preparing the object compound (I) are explained in detail in the following.

Process 1

The object compound (I) or a salt thereof can be prepared by reacting the compound (II) or its reactive derivative at the amino group or a salt thereof with the compound (III) or its reactive derivative at the carboxy group or a salt thereof.

Suitable salts of the compounds (II) and (III) can be referred to the ones as exemplified for the compound (I).

Suitable reactive derivative at the amino group of the compound (II) may include conventional reactive derivative used in amidation, for example, Schiff's base type imino or its tautomeric enamine type isomer formed by the reaction of the compound (II) with a carbonyl compound; a silyl derivative formed by the reaction of the compound (II) with a silyl compound such as bis (trimethylsilyl)acetamide, trimethylsilylacetamide or the like; a derivative formed by reaction of the compound (II) with phosphorus trichloride or phosgene, and the like.

Suitable reactive derivative at the carboxy group of the compound (III) may include an acid halide, an acid anhydride, an activated amide, an activated ester, and the like. The suitable example may be an acid chloride; an acid azide; a mixed acid anhydride with an acid such as substituted phosphoric acid (e.g., dialkylphosphoric acid, phenylphosphoric acid, diphenylphosphoric acid, dibenzylphosphoric acid, halogenated

phosphoric acid, etc.), dialkylphosphorous acid, sulfurous acid, alkanesulfonic acid (e.g., methanesulfonic acid, ethanesulfonic acid, etc.), thiosulfuric acid, sulfuric acid, alkylcarbonic acid, aliphatic carboxylic acid (e.g., pivalic acid, pentanoic acid, isopentanoic acid,

2-ethylbutyric acid, acetic acid or trichloroacetic acid, etc.) or aromatic carboxylic acid (e.g., benzoic acid, etc.); a symmetrical acid anhydride; an activated amide with imidazole, dimethylpyrazole, triazole or tetrazole; or an activated ester (e.g., syanomethyl ester, methoxymethyl ester, dimethyliminomethyl $[(CH_3)_2\overset{+}{N} = CH-]$ ester, vinyl ester, propargyl ester, p-nitrophenyl ester, 2,4-dinitrophenyl ester, trichlorophenyl ester, pentachlorophenyl ester, mesyl phenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester, or an ester with N,N-dimethylhydroxylamine, 1-hydroxy-2-(1H)-pyridone, N-hydroxysuccinimide, N-hydroxyphthalimide or 1-hydroxy-6-chloro-1H-benzotriazole) and the like. These reactive derivatives can be optionally selected from them according to the kind of the compound (III) to be used.

The reaction is usually carried out in a conventional solvent such as water, acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction. Among these solvents, hydrophilic solvents may be used in a mixture with water.

When the compound (III) is used in free acid form or its salt form in the reaction, the reaction is

preferably carried out in the presence of a conventional condensing agent such as N,N-dicyclohexylcarbodiimide; N-cyclohexyl-N'-morpholinoethylcarbodiimide; N-cyclohexyl-N'-(4-diethylaminocyclohexyl)carbodiimide; N,N-diethylcarbodiimide; N,N-diisopropylcarbodiimide; N-ethyl-N'-(3-dimethylaminopropyl)carbodiimide; N,N-carbonylbis(2-methylimidazole); pentamethylene-ketene-N-cyclohexylimine; diphenylketene-N-cyclohexylimine; ethoxyacetylene; ethyl polyphosphate; isopropyl polyphosphate; diethyl phosphorochloridite; phosphorus oxychloride; phosphorus trichloride; phosphorus pentachloride; thionyl chloride; oxalyl chloride; triphenylphosphine; N-ethyl-7-hydroxybenzisoxazolium fluoroborate; N-ethyl-5-phenylisoxazolium-3'-sulfonate; 1-(p-chlorobenzenesulfonyloxy)-6-chloro-1H-benzotriazole; so-called Vilsmeier reagent, for example (chloromethylene) dimethylammonium chloride produced by the reaction of dimethylformamide with thionyl chloride or phosgene, a compound produced by the reaction of dimethylformamide with phosphorus oxychloride, etc.; or the like.

The reaction may be also carried out in the presence of an inorganic or an organic base such as an alkali metal hydroxide, an alkali metal bicarbonate, alkali metal carbonate, alkali metal acetate, tri(lower)-alkylamine, pyridine, N-(lower)alkylmorpholine, N,N-di-(lower)alkylbenzylamine, N,N-di(lower)alkylaniline, or the like. When the base or the condensing agent is in liquid, it can be used also as a solvent. The reaction temperature is not critical, and the reaction is usually carried out under cooling or at ambient temperature.

Process 2 :

The object compound (Ib) or a salt thereof can be prepared by subjecting the compound (Ia) or a salt thereof

to elimination reaction of the carboxy protective group on $R_a^2$.

Suitable salts of the compounds (Ia) and(Ib) can be referred to the ones as exemplified for the compound (I).

The present reaction is carried out in accordance with a conventional method such as hydrolysis, reduction or the like.

In case that the protective group is an ester, the protective group can be eliminated by hydrolysis. Hydrolysis is preferably carried out in the presence of a base or an acid. Suitable base may include an inorganic base and an organic base such as an alkali metal (e.g. sodium, potassium, etc.), an alkaline earth metal (e.g. magnesium, calcium, etc.), the hydroxide or carbonate or bicarbonate thereof, trialkylamine (e.g. trimethylamine, triethylamine, etc.), picoline, 1,5-diazabicyclo[4,3,0]none-5-ene, 1,4-diazabicyclo[2,2,2]octane, 1,8-diazabicyclo[5,4,0]undecene-7, or the like. Suitable acid may include an organic acid (e.g. formic acid, acetic acid, propionic acid, trifluoroacetic acid, etc.) and an inorganic acid (e.g. hydrochloric acid, hydrobromic acid, sulfuric acid, etc.). The acidic hydrolysis using trifluoroacetic acid is usually accelerated by addition of anisole.

The reaction is usually carried out in a solvent such as water, methylene chloride, an alcohol (e.g. methanol, ethanol, etc.), a mixture thereof or any other solvent which does not adversely influence the reaction. A liquid base or acid can be also used as the solvent. The reaction temperature is not critical and the reaction is usually carried out under cooling to warming.

Reduction can be applied preferably for elimination of the protective group such as 4-nitrobenzyl, 2-iodoethyl, 2,2,2-trichloroethyl, or the like.

Reduction is carried out in a conventional manner, including chemical reduction and catalytic reduction.

Suitable reducing agents to be used in chemical reduction are a combination of a metal (e.g. tin, zinc, iron, etc.) or metallic compound (e.g. chromium chloride, chromium acetate, etc.) and an organic or inorganic acid (e.g. formic acid, acetic acid, propionic acid, trifluoroacetic acid, p-toluenesulfonic acid, hydrochloric acid, hydrobromic acid, etc.).

Suitable catalysts to be used in catalytic reduction are conventional ones such as platinum catalysts (e.g. platinum plate, spongy platinum, platinum black, colloidal platinum, platinum oxide, platinum wire, etc.), palladium catalysts (e.g. spongy palladium, palladium black, palladium oxide, palladium on carbon, colloidal palladium, palladium on barium sulfate, palladium on barium carbonate, etc.), nickel catalysts (e.g. reduced nickel, nickel oxide, Raney nickel, etc.), cobalt catalysts (e.g. reduced cobalt, Raney cobalt, etc.), iron catalysts (e.g. reduced iron, Raney iron, etc.), copper catalysts (e.g. reduced copper, Raney copper, Ullman copper, etc.) and the like.

The reduction is usually carried out in a solvent such as water, alcohol (e.g. methanol, ethanol, etc.), N,N-dimethylformamide, tetrahydrofuran, a mixture thereof or any other solvent which does not adversely affect the reaction.
Additionally, in case that the above-mentioned acids to be used in chemical reduction are in liquid, they can also be used as a solvent.

The reaction temperature of this reduction is not critical and the reaction is usually carried out under cooling to warming.

The object compound (I) and pharmaceutically acceptable salts thereof of the present invention are

novel compounds which exhibit high antibacterial activity and inhibit the growth of a wide variety of pathogenic microorganisms including Gram-positive and Gram-negative bacteria and are useful as antimicrobial agents. For therapeutic purpose, the compounds according to the present invention can be used in the form of conventional pharmaceutical preparation which contain said compounds, as an active ingredient, in admixture with a pharmaceutically acceptable carrier such as an organic or an inorganic solid or liquid excipient suitable for oral, parenteral or external administration. The pharmaceutical preparations may be in solid form such as capsule, tablet, dragee, ointment or suppository, or in liquid form such as solution, suspension, or emulsion. If desired, there may be included in the above preparations auxiliary substances, stabilizing agents, wetting or emulsifying agents, buffers and other commonly used additives, such as lactose, fumaric acid, citric acid, tartaric acid, stearic acid, maleic acid, succinic acid, malic acid, magnesium stearate, terra alba, sucrose, corn starch, talc, gelatin, agar, pectin, peanut oil, olive oil, cacao butter, ethylene glycol and the like.

While the dosage of the compounds will vary depending upon the age and condition of the patient, an average single dose of about 10 mg, 50 mg, 100 mg, 250 mg, 500 mg, and 1000 mg of the compounds according to the present invention was proved to be effective for treating infectious diseases coused by pathogenic bacteria. In general, amounts between 1 mg/body and about 6,000 mg/body or even more may be administered per day.

Now, in order to show the utility of the object compound (I), with regard to a representative compound of this inventions, the test data on the in vitro antibacterial activity are shown in the following.

Test Compound

Sodium 7-[2-(5-amino-1,2,4-thiadiazol-3-yl)-2-carboxymethoxyiminoacetamido]-3-[(4-carboxy-3-hydroxyisothiazol-5-yl)thiomethyl]-3-cephem-4-carboxylate (syn isomer)

Test Method

In vitro antibacterial activity was determined by the two-fold agar-plate dilution method as described below.

One loopful of an overnight culture of each test strain in Trypticase-soy broth ($10^8$ viable cells per ml.) was streaked on heart infusion agar (HI-agar) containing graded concentrations of test compound , and minimal inhibitory concentration (MIC) was expressed in terms of µg/ml after incubation at 37°C for 20 hours.

Test Result

MIC (µg/ml)

| Compound / Test strains | Test Compound |
|---|---|
| Proteus mirabilis 18 | <0.025 |
| Proteus vulgaris 2 | <0.025 |

The following Preparations and Examples are given for the purpose of illustrating the present invention.

Preparation 1

To a mixture of 2-(5-formamido-1,2,4-thiadiazol-3-yl)-2-hydroxyiminoacetic acid (syn isomer) (9.60 g) and potassium t-butoxide (14.9 g) in dimethylsulfoxide (100 ml) was added a solution of t-butyl 2-bromo-2-methylbutyrate (14.9 g) in dimethylsulfoxide (10 ml) below 25°C under stirring, which was continued for 3 days at room temperature. The reaction mixture was poured into a mixture of concentrated hydrochloric acid (7.4 ml), cold water (500 ml) and diethyl ether (200 ml) under stirring and the mixture was adjusted to pH 6.5 with aqueous solution of sodium bicarbonate. The aqueous layer was separated out, diethyl ether was added thereto and the mixture was adjusted to pH 2.0 with 6N hydrochloric acid. The organic layer was separated out and the aqueous layer was extracted with diethyl ether. The organic layer and the extract were combined, washed with brine, dried over magnesium sulfate and evaporated to dryness. The residue was crystallized with diethyl ether, collected by filtration and dried to give 2-(5-formamido-1,2,4-thiadiazol-3-yl)-2-(1-t-butoxycarbonyl-1-methylpropoxyimino)acetic acid (syn isomer) (2.76 g) and the same compound was also obtained from the filtrate.

mp 128 to 131°C (dec.)

IR (Nujol) : 3550, 3150, 3020, 1730, 1670, 1540, 1250, 1140, 1110 $cm^{-1}$

NMR ($DMSO-d_6+D_2O$, δ) : 0.88 (3H, t, J=7Hz), 1.38 (3H, s), 1.43 (9H, s), 1.86 (2H, q, J=7Hz), 8.82 (1H, s)

Preparation 2

A solution of 2-(5-formamido-1,2,4-thiadiazol-3-yl)-2-(1-t-butoxycarbonyl-1-methylpropoxyimino)acetic acid (syn isomer) (3.58 g) in 1N aqueous solution (22.4 ml) of sodium hydroxide was stirred for 24 hours at room

temperature and ethyl acetate (30 ml) was added thereto. The mixture was adjusted to pH 2.0 with 1N hydrochloric acid, the organic layer was separated out and the aqueous layer was extracted with ethyl acetate. The organic layer and the extract were combined, washed with water, dried over magnesium sulfate and concentrated to give semisolid, which was triturated with diisopropyl ether to give 2-(5-amino-1,2,4-thiadiazol-3-yl)-2-(1-t-butoxycarbonyl-1-methylpropoxyimino)acetic acid (syn isomer) (2.2 g).

mp 187 to 189°C (dec.)

IR (Nujol) : 3420, 3320, 3220, 1750, 1720, 1635, 1535, 1420, 1240, 1140, 1020 $cm^{-1}$

NMR ($DMSO-d_6$, δ) : 0.85 (3H, t, J=7Hz), 1.40 (3H, s), 1.43 (9H, s), 1.86 (2H, q, J=7Hz), 8.21 (2H, broad s)

Example 1

To a solution of 7-amino-3-[(4-carboxy-3-hydroxyisothiazol-5-yl)thiomethyl]-3-cephem-4-carboxylic acid (1.0 g) and sodium bicarbonate (648 mg) in 50% aqueous solution (26 ml) of acetone was added 2-(5-amino-1,2,4-thiadiazol-3-yl)-2-carboxymethoxyiminoacetic methanesulfonic anhydride (syn isomer) (832 mg) at 10°C under stirring, which was continued for 30 minutes at 10 to 15°C adjusting to pH 7 with an aqueous solution of sodium bicarbonate.

To the reaction mixture was added ethyl acetate (20 ml) and the mixture was adjusted to pH 1.5 with 6N hydrochloric acid. After an insoluble material was filtered off, the organic layer was separated out and evaporated to dryness. The residue was triturated in acetonitrile to give a powder (650 mg). The powder was dissolved in methanol (20 ml) and a solution of sodium acetate (82 mg) in methanol (2 ml) was added thereto.

The resulting precipitate was filtered off and the filtrate was concentrated to 5 ml. The resulting precipitate was collected by filtration, washed with ethanol and dried to give sodium 7-[2-(5-amino-1,2,4-thiadiazol-3-yl)-2-carboxymethoxyiminoacetamido]-3-[(4-carboxy-3-hydroxyisothiazol-5-yl)thiomethyl]-3-cephem-4-carboxylate (syn isomer) (230 mg).

mp 205 to 210°C (dec.)

IR (Nujol) : 3300, 1760, 1680, 1600, 1520, 1240, 1050, 1020 $cm^{-1}$

NMR ($D_2O$, δ) : 3.53 and 3.77 (2H, ABq, J=18Hz), 4.02 and 4.43 (2H, ABq, J=14Hz), 4.78 (2H, s), 5.23 (1H, d, J=4Hz), 5.90 (1H, d, J=4Hz)

Example 2

The following compound was obtained by reacting 7-amino-3-[(4-carboxy-3-hydroxyisothiazol-5-yl)thiomethyl]-3-cephem-4-carboxylic acid with 2-(5-amino-1,2,4-thiadiazol-3-yl)-2-ethoxyiminoacetyl chloride hydrochloride (syn isomer) according to a similar manner to that of Example 1.

7-[2-(5-Amino-1,2,4-thiadiazol-3-yl)-2-ethoxyiminoacetamido]-3-[(4-carboxy-3-hydroxyisothiazol-5-yl)thiomethyl]-3-cephem-4-carboxylic acid (syn isomer)

mp 180 to 185°c (dec.)

IR (Nujol) : 3400, 3250, 3200, 1770, 1700, 1670, 1620, 1540 $cm^{-1}$

NMR ($D_2O$+$NaHCO_3$, δ) : 5.88 (1H, d, J=4Hz), 5.25 (1H, d, J=4Hz), 4.40 (2H, q, J=7Hz), 4.36 and 3.93 (2H, ABq, J=14Hz), 3.80 and 3.53 (2H, ABq, J=18Hz), 1.37 (3H, t, J=7Hz)

Example 3

The following compound was obtained by reacting 7-amino-3-[(4-carboxy-3-hydroxyisothiazol-5-yl)thiomethyl]-3-cephem-4-carboxylic acid with 2-(5-amino-1,2,4-thiadiazol-3-yl)-2-cyclopentyloxyiminoacetyl chloride hydrochloride (syn isomer) according to a similar manner to that of Example 1.

7-[2-(5-Amino-1,2,4-thiadiazol-3-yl)-2-cyclopentyloxyiminoacetamido]-3-[(4-carboxy-3-hydroxyisothiazol-5-yl)thiomethyl]-3-cephem-4-carboxylic acid (syn isomer).

mp 182 to 187°C (dec.)

IR (Nujol) : 3300, 3200, 1770, 1700, 1680, 1610, 1520, 1400, 1240, 1000cm$^{-1}$

NMR(DMSO-$d_6$, δ) : 9.47 (1H, d, J=8Hz), 8.10 (2H, broad s), 5.80 (1H, dd, J=8Hz, 4Hz), 5.17 (1H, d, J=4Hz), 4.8-4.6 (1H) 4.27 and 4.07 (2H, ABq, J=14Hz), 3.67 (2H, broad s), 2.0-1.3 (8H)

Example 4

The following compound was obtained according to a similar manner to that of Example 1.

7-[2-(5-Amino-1,2,4-thiadiazol-3-yl)-2-(1-carboxy-1-methylpropoxyimino)acetamido]-3-[(4-carboxy-3-hydroxyisothiazol-5-yl)thiomethyl]-3-cephem-4-carboxylic acid (syn isomer).

mp 207 to 210°C (dec.)

IR (Nujol) : 3300, 3200, 1770, 1730-1670, 1620, 1520, 1240, 1160, 1140, 1000 cm$^{-1}$

Example 5

To a solution of phosphorus pentachloride (1.33 g) in methylene chloride (21 ml) was added 2-(5-amino-1,2,4-thiadiazol-3-yl)-2-(1-t-butoxycarbonyl-1-methylpropoxyimino)acetic acid (syn isomer) (2.10 g) at -15°C under stirring, which was continued for 40 minutes

at -20 to -12°C. The reaction mixture was added to a cold solution of 7-amino-3-[(4-carboxy-3-hydroxyisothiazol-5-yl)thiomethyl]-3-cephem-4-carboxylic acid (2.61 g) and trimethylsilylacetamide (13.5 g) in methylene chloride (26 ml) at -20°C under stirring, which was continued for 40 minutes at -20 to -10°C. The reaction mixture was poured into a solution of sodium bicarbonate (3.8 g) in water (80 ml), the aqueous layer was separated out and washed with ethyl acetate. To the aqueous soltuion was added ethyl acetate and the mixture was adjusted to pH 1.5 with 6N hydrochloric acid. The organic layer was separated and the aqueous layer was extracted with ethyl acetate. The organic layer and the extract were combined, washed with water, dried over magnesium sulfate and concentrated to a small volume. The resulting precipitate was collected by filtration and dried to give 7-[2-(5-amino-1,2,4-thiadiazol-3-yl)-2-(1-t-butoxycarbonyl-1-methylpropoxyimino)-acetamido]-3-[(4-carboxy-3-hydroxyisothiazol-5-yl)-thiomethyl]-3-cephem-4-carboxylic acid (syn isomer) (1.97 g).

mp 203 to 210°C (dec.)

IR (Nujol) : 3300, 3200, 1780, 1720, 1680, 1615, 1515, 1250, 1140 $cm^{-1}$

NMR ($DMSO-d_6+D_2O$, δ) : 0.88 (3H, t, J=7Hz), 1.40 (12H, s), 1.95 (2H, q, J=7Hz), 3.50 and 3.87 (2H, ABq, J=18Hz), 3.9-4.5 (2H, m), 5.18 (1H, d, J=5Hz), 5.85 (1H, d, J=5Hz)

Example 6

A mixture of 7-[2-(5-amino-1,2,4-thiadiazol-3-yl)-2-(1-t- butoxycarbonyl-1-methylpropoxyimino)acetamido]-3-[(4-carboxy-3-hydroxyisothiazol-5-yl)thiomethyl]-3-cephem-4-carboxylic acid (syn isomer) (1.90 g) and anisole (2 ml) in trifluoroacetic acid (10 ml) was stirred for one hour under cooling in an ice bath.

The reaction mixture was poured into cold diisopropyl ether (60 ml) and the resulting precipitate was collected by filtration. The precipitate was dissolved in a solution of sodium bicarbonate (892 mg) in water (40 ml), washed with ethyl acetate and a mixture of ethyl acetate (25 ml) and tetrahydrofuran (25 ml) was added thereto. The mixture was adjusted to pH 4.5 with 6N hydrochloric acid and the aqueous layer was separated out. The aqueous solution was adjusted to pH 3 with 6N hydrochloric acid and extracted with a mixed solvent of ethyl acetate and tetrahydrofuran (1:1). The extract was washed with brine, dried over magnesium sulfate and concentrated to small volume. The resulting precipitate was collected by filtration, washed with diethyl ether and dried to give 7-[2-(5-amino-1,2,4-thiadiazol-3-yl)-2-(1-carboxy-1-methylpropoxyimino)acetamido]-3-[(4-carboxy-3-hydroxyisothiazol-5-yl)thiomethyl]-3-cephem-4-carboxylic acid (syn isomer) (1.03 g).

mp 207 to 210°C (dec.)

IR (Nujol) : 3300, 3200, 1770, 1730-1670, 1620, 1520, 1240, 1160, 1140, 1000 $cm^{-1}$

NMR ($D_2O$ + $NaHCO_3$, $\delta$) : 0.92 (3H, t, J=7Hz), 1.53 (3H, s), 1.95 (2H, q, J=7Hz), 3.45 and 3.90 (2H, ABq, J=18Hz), 3.90 and 4.40 (2H, ABq, J=14Hz), 5.24 (1H, d, J=5Hz), 5.87 (1H, d, J=5Hz)

Example 7

The following compound was obtained according to a similar manner to that of Example 6.

Sodium 7-[2-(5-amino-1,2,4-thiadiazol-3-yl)-2-carboxymethoxyiminoacetamido]-3-[(4-carboxy-3-hydroxyisothiazol-5-yl)thiomethyl]-3-cephem-4-carboxylate (syn isomer).

mp 205 to 210°C (dec.)

IR (Nujol) : 3300, 1760, 1680, 1600, 1520, 1240, 1050, 1020 $cm^{-1}$

What we claim is :

1. A compound of the formula :

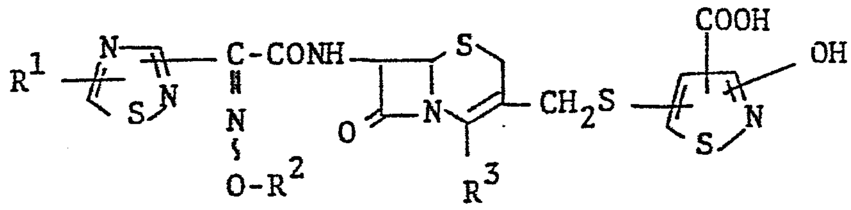

wherein $R^1$ is amino or a protected amino,
$R^2$ is lower alkyl, carboxy(lower)alkyl, protected carboxy(lower)alkyl or cycloalkyl, and
$R^3$ is carboxy or a protected carboxy,
and pharmaceutically acceptable salts thereof.

2. A compound of claim 1,
wherein $R^1$ is amino or acylamino,
$R^2$ is lower alkyl, carboxy(lower)alkyl, esterified carboxy(lower)alkyl or cycloalkyl, and
$R^3$ is carboxy or esterified carboxy.

3. Syn isomer of a compound of claim 2.

4. A compound of claim 3,
wherein $R^1$ is amino,
$R^2$ is lower alkyl, carboxy(lower)alkyl, lower alkoxycarbonyl(lower)alkyl or cycloalkyl, and
$R^3$ is carboxy.

5. A compound of claim 4, which is selected from the group consisting of :

sodium 7-[2-(5-amino-1,2,4-thiadiazol-3-yl)-2-carboxymethoxyiminoacetamido]-3-[4-carboxy-3-hydroxyisothizol-5-yl)thiomethyl]-3-cephem-4-carboxylate (syn isomer),

7-[2-(5-amino-1,2,4-thiadiazol-3-yl)-2-ethoxyimino-acetamido]-3-[(4-carboxy-3-hydroxyisothiazol-5-yl)thiomethyl]-3-cephem-4-carboxylic acid (syn isomer),

7-[2-(5-amino-1,2,4-thiadiazol-3-yl)-2-cyclopentyloxy-iminoacetamido]-3-[(4-carboxy-3-hydroxyisothiazol-5-yl)thiomethyl]-3-cephem-4-carboxylic acid (syn isomer),

7-[2-(5-amino-1,2,4- thiadiazol-3-yl)-2-(1-t-butoxycarbonyl-1-methylpropoxyimino)-acetamido]-3-[(4-carboxy-3-hydroxyisothiazol-5-yl)-thiomethyl]-3-cephem-4-carboxylic acid (syn isomer), and

7-[2-(5-amino-1,2,4-thiadiazol-3-yl)-2-(1-carboxy-1-methylpropoxyimino)acetamido]-3-[(4-carboxy-3-hydroxyisothiazol-5-yl)thiomethyl]-3-cephem-4-carboxylic acid (syn isomer).

6. A process for preparing a compound of the formula :

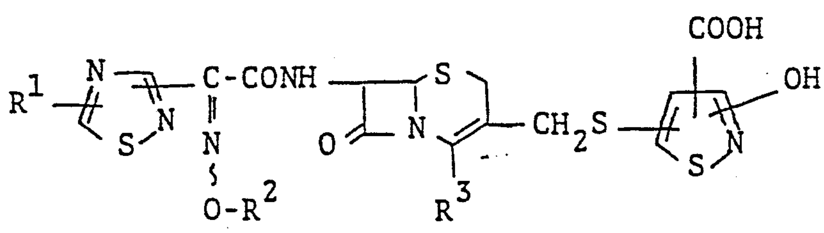

wherein $R^1$ is amino or a protected amino,
$R^2$ is lower alkyl, carboxy(lower)alkyl, protected carboxy(lower)alkyl or cycloalkyl, and
$R^3$ is carboxy or a protected carboxy,
or a salt thereof,
which comprises,

(1) reacting a compound of the formula :

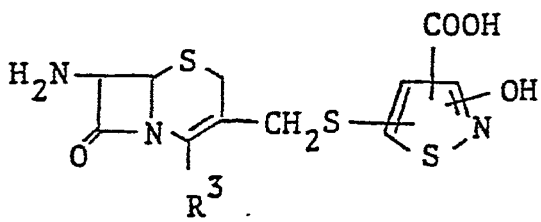

wherein $R^3$ is as defined above,
or its reactive derivative at the amino group or a salt thereof, with a compound of the formula :

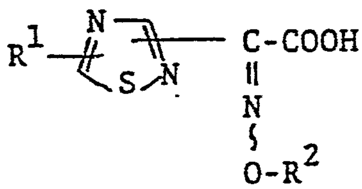

wherein $R^1$ and $R^2$ are each as defined above,
or its reactive derivative at the carboxy group or a salt thereof to give a compound of the formula :

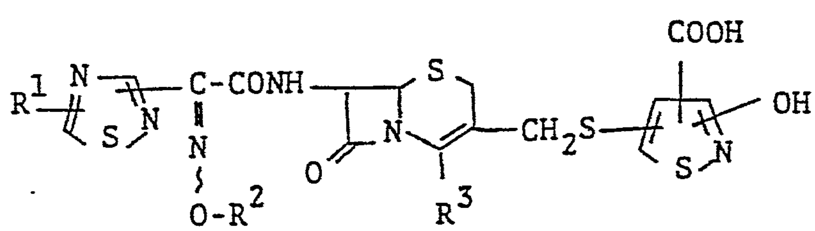

wherein $R^1$, $R^2$ and $R^3$ are each as defined above, or a salt thereof, or

(2) subjecting a compound of the formula :

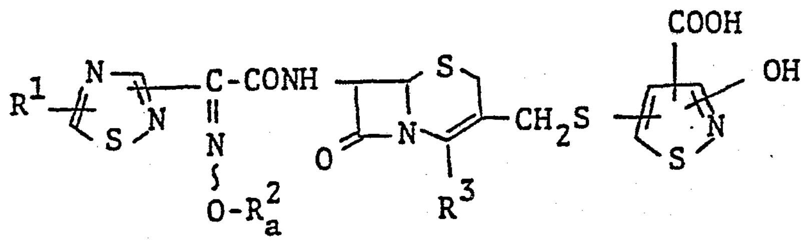

wherein $R^1$ and $R^3$ are each as defined above, and $R^2_a$ is protected carboxy(lower)alkyl, or a salt thereof to elimination reaction of the carboxy protective group on $R^2_a$ to give a compound of the formula :

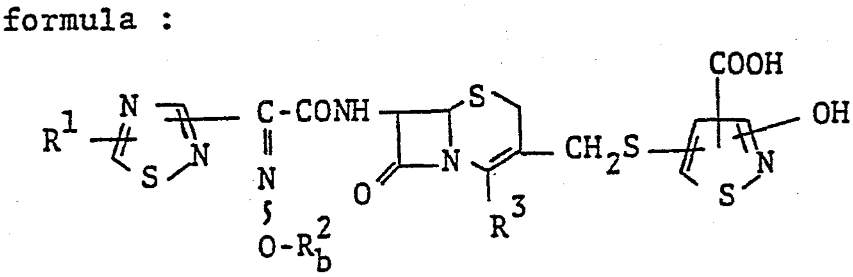

wherein $R^1$ and $R^3$ are each as defined above, and $R^2_b$ is carboxy(lower)alkyl, or a salt thereof.

7. A pharmaceutical composition comprising an effective amount of a compound of claim 1 or pharmaceutically acceptable salt thereof in association with a pharmaceutically acceptable, substantially non-toxic carrier or excipient.

8. A method for producing a pharmaceutical composition which comprises mixing a compound of claim 1 or pharmaceutically acceptable salt thereof as an active ingredient with an inert carrier.

9. Use of the compound of claim 1 or pharmaceutically acceptable salt thereof for treatment of infectious diseases in human being and animals.